(19) Europäisches Patentamt
European Patent Office
Office européen des brevets

(11) Numéro de publication : 0 298 803 B1

(12) # FASCICULE DE BREVET EUROPEEN

(45) Date de publication du fascicule du brevet : 04.09.91 Bulletin 91/36

(21) Numéro de dépôt : 88401507.4

(22) Date de dépôt : 17.06.88

(51) Int. Cl.$^5$ : C07B 37/04, C07C 211/00, C07C 22/00, C07C 43/225, C07C 39/24, C07D 213/26, C07D 207/32, C07D 215/26, C07D 333/12, C07D 307/38, C07C 209/00

(54) **Procédé de perhalogénoalkylation de dérivés aromatiques.**

(30) Priorité : 23.06.87 FR 8709001

(43) Date de publication de la demande : 11.01.89 Bulletin 89/02

(45) Mention de la délivrance du brevet : 04.09.91 Bulletin 91/36

(84) Etats contractants désignés : AT BE CH DE ES FR GB GR IT LI LU NL SE

(56) Documents cités :
EP-A- 0 114 359
EP-A- 0 206 951

(73) Titulaire : RHONE-POULENC CHIMIE
25, quai Paul Doumer
F-92408 Courbevoie Cédex (FR)

(72) Inventeur : Tordeux Marc
1, rue Seignelay
F-92330 - Sceaux (FR)
Inventeur : Wakselman Claude
5, rue Chanez
F-75016 - Paris (FR)
Inventeur : Langlois Bernard
91, rue Duguesclin
F-69006 - Lyon (FR)

(74) Mandataire : Le Pennec, Magali et al
RHONE-POULENC INTERSERVICES Service Brevets Chimie 25, Quai Paul Doumer
F-92408 Courbevoie Cédex (FR)

EP 0 298 803 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé de perhalogéno alkylation de dérivés aromatiques. Elle concerne plus particulièrement un procédé de perhalogénoalkylation de dérivés aromatiques par des halogénures de perfluoroalkyle ou de perhalogénoalkyle trifluorométhylés.

Il est connu, d'après J. FUCHIKAMI, I. OJIMA (J. Fluorine Chemistry 1983, 22, 541) de perfluorer des amines aromatiques ou des phénols par des iodures ou des bromures de perfluoroalkyle dont la chaîne alkyle contient au moins trois atomes de carbone en présence de cuivre métallique comme catalyseur. La trifluorométhylation de la parachloroaniline à l'aide de l'iodure de trifluorométhyle ne donne que des traces de trifluorométhylparachloroaniline. L'iodure de trifluorométhyle n'étant pas industriel, le rendement de la réaction étant très faible cette méthode n'est pas transposable au stade industriel. La trifluorométhylation des phénols n'est pas décrite.

Il est également connu d'après le brevet européen N° 114359 de perfluoroalkyler des dérivés aromatiques et en particulier l'anisole (exemple 9) à l'aide d'iodure de perfluoroalkyle à longue chaîne, pendant 30 heures à 170°C et en présence d'un catalyseur à base de ruthénium. La trifluorométhylation n'est jamais décrite dans ce brevet comme dans l'article précédent. L'iodure de trifluorométhyle n'étant pas industriel, cette méthode de perfluoroalkylation n'est pas envisageable.

Il est connu d'après la demande de brevet déposée en Europe sous le n° 864201306 et non publiée à ce jour une méthode de perfluoroalkylation de dérivés aromatiques qui consiste dans une première étape à mettre en présence un dérivé aromatique, du dioxyde de soufre, un métal choisi parmi le zinc, l'aluminium, le manganèse, le cadmium, le fer, le magnésium, l'étain, le nickel ou le cobalt dans un solvant aprotique polaire et dans une deuxième étape à ajouter un bromure ou un iodure de perfluoroalkyle.

Dans ce dernier procédé on préfère, parmi les métaux, utiliser le zinc. Parmi les dérivés à alkyler on peut choisir, les amines, les phénols, le benzène, les halogénobenzènes, les alkylbenzènes, les alcoxybenzènes, les composés aromatiques polycycliques ou hétérocycliques éventuellement substitués par un groupe aryloxy, alkyloxy, alkylthio, arylthio, amino, hydroxy, carboxylate, acyloxy, ester, amido, nitrile, acide.

Dans ce même procédé parmi les bromures ou iodures de perfluoroalkyle on préfère utiliser le bromure de perfluoroalkyle lorsque la chaîne alkyle contient un seul atome de carbone et les iodures de perfluoroalkyle lorsque la chaîne alkyle contient au moins deux atomes de carbone.

En effet, le bromure de trifluorométhyle est un gaz extincteur (M.R.C. "GERSTENBERGER, A. HAAS Angew. Cem. Int. "Ed 1981, 20, 647) qui est un produit industriel fabriqué à large échelle, donc d'un coût tout à fait accessible pour l'industrie. L'iodure de trifluorométhyle n'étant pas industriel n'est disponible qu'à un prix qui le rend tout à fait inutilisable. Par contre, dès que la chaîne alkyle a au moins 2 atomes, les iodures de perfluoroalkyle se présentent sur le marché à des prix nettement inférieurs à ceux de leurs homologues bromés.

Ce dernier procédé permet d'atteindre de manière originale les dérivés aromatiques perfluoroalkylés qui se trouvent dans un milieu contenant une quantité importante de sels de zinc. Le zinc présente l'inconvénient d'attaquer le revêtement du réacteur et d'autre part est très difficile à séparer du dérivé aromatique perfluoroalkylé.

La présente invention a permis d'atteindre un procédé tout à fait original de synthèse des dérivés aromatiques perhalogénoalkylés en permettant d'éviter l'usage du zinc métallique.

Elle a pour objet un procédé de perhalogénoalkylation de dérivés aromatiques caractérisé en ce que l'on met en présence, dans un solvant polaire, un dérivé aromatique avec un dithionite ou un hydroxyméthane sulfinate alcalin, alcalinoterreux ou métallique et un halogénure de perfluoroalkyle ou un halogénure de perhalogénoalkyle trifluorométhylé en ce que le dérivé aromatique répond à la formule générale (I) :

$$Ar \text{———} (R)_n \qquad (I)$$

dans laquelle :

— Ar représente un radical aromatique mono ou polycyclique ou hétérocyclique

— R représente au moins un substituant choisi parmi l'hydrogène, le chlore, le brome, les radicaux alkyle linéaires, ramifiés, cycliques saturés ou insaturés éventuellement substitués, alcoxy éventuellement substitué, aryl, éventuellement substitué, aryloxy, alkylthio, arylthio, amino, hydroxy, carboxylate, acyloxy, ester, amido, nitrile, acide.

— n est un nombre entier égal à 1, 2 ou 3.

Pour une meilleure mise en oeuvre de l'invention on préfère utiliser des dérivés aromatiques de formule (I) dans lesquels Ar représente un radical aromatique monocyclique et $R_1$ représente au moins un substituant choisi parmi les radicaux amino ou hydroxy.

Parmi les dérivés aromatiques de formule (I) utilisées dans le procédé de l'invention on peut citer, mais de façon non limitative : le benzène, le toluène, le métaxylène, l'oxyde de phényle, le biphényle, le bromobenzène, le chlorobenzène, l'alcool benzylique, le phényl acétate d'éthyle, la pyridine, la méthyl-2 pyridine ; les amines telles que notamment l'aniline, les méthylanilines, les phénoxyanilines, l'aminonaphtalène, les diaminobenzènes, l'amino-3 pyridine ; les dérivés phénoliques parmi lesquels on peut citer les phénols, les crésols, les phénylphénols, les chlorophénols, les aminophénols, les anisoles, les méthoxyphénols, les dihydroxybenzènes, le terbutyl-4 phénol, le terbutyl-3 phénol, l'hydroxy-2 pyridine.

Selon le procédé de l'invention, l'halogénure de perfluoroalkyle ou de perhalogénoalkyle trifluorométhylé répond de préférence à la formule générale (II) suivante

$$CF_3\text{-}A\ X \quad (II)$$

dans laquelle :

— X représente un halogène choisi parmi le brome, l'iode ou le chlore, et, lorsque A est une liaison covalente, X est limité à l'iode ou au brome.

— A représente une liaison covalente, un groupe $(CF_2)_n$ où n est un nombre entier compris entre 1 et 12 ou un groupe —$CCl_2$—.

D'un point de vue économique on préfère utiliser parmi les halogénures de perfluoroalkyle (A représente $(CF_2)_n$ ou une liaison covalente) le bromure de trifluorométhyle et les iodures de perfluoroalkyle dont la chaîne alkyle contient plus de 1 atome de carbone, et parmi les halogénures de perhalogénoalkyle trifluorométhylés le trichloro 1,1,1 trifluoroéthane.

Le solvant choisi doit, autant que possible, permettre de solubiliser le dithionite ou l'hydroxyméthanesulfinate et l'halogènure de perfluoroalkyle ou de perhalogénoalkyle trifluorométhylé.

Répondent à cette condition, les solvants polaires et parmi eux préférentiellement :

— l'acétonitrile
— le formamide
— le diméthylformamide (D.M.F.)
— le diméthylacétamide (D.M.A.)
— l'hexaméthylphosphoramide (H.M.P.A.)
— la N-méthylpyrrolidone (N.M.P.)
— le diméthylsulfoxyde (DMSO)
— le sulfolane

Parmi les amides on préfère utiliser notamment : le formamide, le diméthylformamide, la N-méthylpyrrolidone, le diméthylacétamide. On préfère tout particulièrement utiliser le diméthylformamide.

Le dithionite alcalin, alcalino terreux ou métallique répond à la formule générale (III) $M(S_2O_4)_n$ dans laquelle n est égal à 1 ou 2 suivant la valence du métal M.

Parmi les composés de formule (III), on préfère utiliser le dithionite de sodium ou de potassium. On préfère tout particulièrement utiliser le dithionite de sodium. Parmi les hydroxyméthanesulfinates, on préfère utiliser l'hydroxyméthanesulfinate de sodium (plus connu sous le nom commercial de Rongalite) ou l'hydroxyméthanesulfinate de zinc (plus connu sous le nom commercial de Decroline®) ou encore l'hydroxyméthanesulfinate de cuivre.

La quantité de dithionite ou d'hydroxyméthane sulfinate introduite est de préférence d'au moins 10% en moles calculé par rapport au composé de formule 1 et encore plus préférentiellement d'environ 100%.

Lorsque l'on utilise un dithionite de formule générale (III) ou un hydroxyméthanesulfinate on ajoute avantageusement une base choisie parmi les hydroxydes alcalins ou alcalino-terreux, l'ammoniaque, la tris-dioxa-3,6 heptylamine, le chlorure de triéthylbenzylammonium, les sels d'acides faibles tels que par exemple le phosphate disodique, le métabisulfite de sodium, l'hydrogénosulfite de sodium, le borate de sodium. On préfère utiliser le phosphate disodique. La quantité de base utilisée varie avantageusement selon un rapport molaire base/dérivé aromatique compris entre 0,3 et 3.

Parmi les perhalogénométhanes on préfère utiliser les perhalogénométhanes gazeux et parmi eux notamment le bromotrifluoro méthane, le bromochlorodifluorométhane, le dichlorodifluorométhane ou les perhalogénométhanes liquides et parmi eux, le trichlorofluorométhane ou le dibromodifluorométhane. Dans la classe des perhalogénométhanes gazeux, on préfère tout particulièrement utiliser le bromotrifluorométhane, moins onéreux car il fait partie des produits industriels utilisés notamment dans la lutte contre l'incendie. En effet ce composé est réputé pour son inertie chimique et il est particulièrement surprenant qu'en présence d'agents libérateurs de radical anion sulfinique il réagisse avec le composé aromatique.

Selon un premier procédé de mise en oeuvre de l'invention avec le dithionite alcalin, celui-ci est introduit dans le réacteur en solution saturée dans l'eau ou le formamide. Il est même possible d'introduire le dithionite

sous forme solide. On préfère éliminer tout l'oxygène présent dans le réacteur puis éventuellement on introduit du dioxyde de soufre et on introduit le perhalogénoalkane.

Selon un deuxième procédé de mise en oeuvre de l'invention avec un hydroxyméthanesulfinate, on introduit ce dernier directement sous forme solide dans le solvant réactionnel. On introduit éventuellement du dioxyde de soufre puis le perhalogénoalkane.

A la fin de la réaction on sépare le ou les solvants et les produits réactionnels puis on purifie le composé aromatique perhalogénoalkylé par extraction à l'aide de solvants tels que l'acétate d'éthyle.

En ce qui concerne les conditions de réactions il est préférable de travailler à une température comprise entre 20° et 85°C et encore plus préférentiellement lorsque l'on utilise un dithionite à une température comprise entre 45°C et 75°C.

Lorsque l'on travaille avec un gaz peu soluble dans le solvant de réaction, la pression réactionnelle est généralement supérieure à 1 bar. Une pression comprise entre 1 et 50 bars est préférée bien que la limite supérieure ne soit pas un critère essentiel mais uniquement préférentiel du point de vue technique.

De préférence le réacteur ne doit pas être constitué d'un matériau réactif tel que ceux décrit dans la demande de brevet publiée sous le numéro EP 165135. On préfère ainsi utiliser un réacteur en verre.

On peut citer parmi les produits obtenus par le procédé de la présente invention : le trifluorométhylbenzène, les trifluorométhyltoluènes, les diméthyltrifluorométhylbenzènes, les triluorométhylanisoles, les trifluorométhylphénoxybenzènes, les trifluorométhylbiphényles, les bromotrifluorométhylbenzènes, les chlorotrifluorométhylbenzènes, les trifluorométhylpyridines,les alcools trifluorométhylbenzyliques, le (trifluorométhylphényl) acétate d'éthyle, les méthyl, méthoxy, chloro, phényl, phénoxytrifluoro méthylanilines, les méthyltrifluorométhylpyridines, les trifluorométhylanilines, les diaminotrifluorométhylbenzènes, les diaminobistrifluorométhylbenzènes, les trifluorométhyl-N,N diéthylanilines, les pentafluoroéthylanilines, les perfluorobutylanilines, les aminotrifluorométhylpyridines, les trifluorométhylphénols, les chlorotrifluorométhylphénols, les méthyltrifluorométhylphénols, les méthoxytrifluorométhylphénols, les aminotrifluorométhylphénols, les terbutyltrifluorométhylphénols, les hydroxytrifluorométhylpyridines.

Les produits objets du procédé de l'invention sont notamment utilisés comme intermédiaires de synthèse dans l'industrie pharmaceutique ou phytosanitaire.

L'invention va être plus complètement décrite à l'aide des exemples suivants quine devront pas être considérés comme limitatifs de l'invention.

EXEMPLE 1

Tous les déplacements chimiques dans les spectres de résonnance magnétique nucléaire sont mesurés par rapport à $CFCl_3$ pris comme référence.

Dans un flacon en verre épais, on place 10 ml d'aniline 50 ml de diméthylformamide, 15 ml d'eau, 10 g de dithionite de sodium et 8 g de phosphate disodique. On fait le vide dans celui-ci, puis on le thermostate à 65°C. Le flacon est agité pendant 2 heures sous une pression en bromotrifluorométhane qui est maintenue entre 3,7 et 2,5 bars. Le flacon est ensuite ouvert, les trifluorométhylanilines sont entraînées à la vapeur, et on obtient après extraction à l'éther et purification :

4,0 g de trifluorométhyl-2 aniline(1) ; Rdt = 23% ; δF = –63 ppm
Eb 14 mm Hg : 66°C
1,9 g de trifluorométhyl-4 aniline(2) ; Rdt = 11% ; δF = –60 ppm
Eb 14 mm Hg : 86°C
1 g de bis-trifluorométhyl-2,4 aniline(3) ; Rdt = 4% ;
δF = –60,8 ppm, –64 ppm ; H = 7,73 ppm (1H, s large)
7,53 ppm (1H, d ; J : 8,5 Hz) 6,67 ppm (1H, d)
0,5 g de bistrifluorométhyl-2,6 aniline(4) ; Rdt = 2%
δF = –63,6 ppm ; H = 7,67 ppm (2H, d ; J = 8,5 Hz)
6,83 ppm (1H, t)

EXEMPLE 2 A 6

On procède comme dans l'exemple 1 en thermostatant à la température indiquée dans le tableau.
Après le temps indiqué dans le tableau on obtient les résultats suivants :

| N° | température °C | temps heures | rendements % | | | | observations |
|---|---|---|---|---|---|---|---|
| | | | (1) ortho | (2) para | (3) 2,6 | (4) 2,4 | |
| 1 | 65° C | 2 | 23 | 11 | 2 | 4 | exemple 1 |
| 2 | 55° C | 3 | 32 | 17 | 2 | 4 | |
| 3 | 45° C | 3 | 25 | 12 | 2 | 4 | |
| 4 | 35° C | 5 | 19 | 9 | 1 | 2 | réaction non terminée |
| 5 | 20° C | 3 | 15 | 7 | | | idem |
| 6 | 65° C | 2 | 21 | 11 | 2 | 3 | eau et phosphate disodique remplacés par 15 ml d'ammoniaque concentrée |

EXEMPLE 7

Dans un flacon en verre épais, on place 10 g de dithionite de sodium dihydrate, 50 ml de diméthylformamide, 15 ml d'ammoniaque et 10 ml de toluène. On fait le vide dans le flacon ; on le thermostate à 65°C. Il est agité pendant 2 heures sous une pression de bromotrifluorométhane qui est maintenue entre 3,7 et 2,5 bars. Le flacon est ensuite ouvert et les solides sont éliminés par filtration. Le mélange est acidifié par de l'acide chlorhydrique à 10% jusqu'à pH = 1. On l'extrait à l'éther et la phase organique est lavée avec de l'acide chlorhydrique à 10 % puis avec des solutions aqueuses de carbonate de sodium et de chlorure de sodium. L'éther est évaporé sous vide et l'analyse du résidu indique la formation de

1,4 g de trifluorométhyl-2 toluène ; Rdt = 8% ; δF = −60,6 ppm
0,9 g de trifluorométyl-3 toluène ; Rdt = 5% ; δF = −61,6 ppm
0,9 g de trifluorométhyl-4 toluène ; Rdt = 5% ; δF = −61,8 ppm

EXEMPLE 8

On procède comme à l'exemple 7 en remplaçant le toluène par 10 ml de benzène. Après 2 heures de réaction et traitement, l'analyse du résidu indique la formation de trifluorométhylbenzène avec un rendement de 22%.

EXEMPLE 9

On procède comme à l'exemple 1 en remplaçant l'aniline par 10 ml d'anisole.

Après 2 heures de réaction, entraînement à la vapeur, extraction à l'éther et évaporation du solvant, le mélange est analysé par résonance magnétique nucléaire du fluor et par chromatographie en phase gazeuse. On obtient :

1) le trifluorométhyl-2 anisole ; Rdt = 14% δF = −60,7 ppm
2) le trifluorométhyl-4 anisole ; Rdt = 7% δF = −61,7 ppm

EXEMPLE 10

On procède comme à l'exemple 7 en remplaçant l'ammoniaque par 10 g de phosphate disodique et 15 ml d'eau.

Après 2 heures de réaction, extraction à l'éther et distillation à pression atmosphérique de l'éther et du toluène.

On obtient un mélange constitué de :
2,8 g de trifluorométhyl-2 toluène ; Rdt = 16% ; δF = −60,6 ppm
0,9 g de trifluorométhyl-3 toluène ; Rdt = 5% ; δF = −61,6 ppm
1,2 g de trifluorométhyl-4 toluène ; Rdt = 7% ; δF = −61,8 ppm

EXEMPLE 11

On procède comme dans l'exemple 1 en remplaçant l'aniline par 9,4 g de phénol.
Après 2 heures de réaction, entraînement à la vapeur et purification, on obtient :
1) 3,4 g de trifluorométhyl-2 phénol (Eb 147-148°C) ; Rdt = 20% ; δF = −61,6 ppm
2) 1,4 g de trifluorométhyl-4 phénol (Eb 183°C) ; Rdt = 8% δF = −60,6 ppm

EXEMPLE 12

On procède comme dans l'exemple 11 en remplaçant l'eau et le phosphate disodique par 15 ml d'ammoniaque concentrée.

Après 2 heures de réaction et purification, on obtient 1,6 g d'un mélange 2,2/1 (mole/mole) de trifluorométhyl-2 et trifluorométhyl-4 phénol ; Rdt total = 10%.

EXEMPLE 13

Dans un erlenmeyer on place 2 g d'hydroxyméthanesulfinate de sodium, 5 ml de diméthylformamide, 0,2 l d'eau, 5,5 g d'iodure de perfluorooctyle et 1 ml d'aniline. On agite pendant 6 heures puis on ajoute 10 g de glace et 5 ml d'acide chlorhydrique concentré. Le mélange est extrait à l'éther. On obtient ainsi un mélange équimoléculaire de perfluorooctyl-2 anilines.
δF = −82 ppm (3F), −110 ppm (2F), −124 ppm (6F), −127 ppm (2F)
δF = −82 ppm (3F), −110,2 ppm (2F), −124 ppm (6F), −127 ppm (2F)

EXEMPLE 14

Dans un erlenmeyer on place 1 g de dithionite de sodium, 1 g de phosphate disodique, 5 ml de diméthylformamide, 1,5 ml d'eau, 1 ml d'aniline et 5,5 g d'iodure de perfluorooctyle. On agite pendant 12 heures ; on ajoute ensuite 10 g de glace et 5 ml d'acide chlorhydrique concentré. Le mélange est extrait à l'éther. Après purification on obtient un mélange équimoléculaire de perfluorooctyl-2 et -4 anilines avec un rendement total de 55%.

EXEMPLE 15

Dans un erlenmeyer on place 1 g de dithionite de sodium, 1g de phosphate disodique, 5 ml de diméthylformamide, 1,5 ml d'eau, 1 g de phénol et 3,5 g d'iodure de perfluorobutyle. On agite pendant 36 heures puis on ajoute 5 ml d'acide chlorhydrique concentré et 10 g de glace. Le mélange est extrait à l'éther. Après purification, on obtient les perfluorobutyl-2 et -4 phénols avec respectivement des rendements de 15 et 19% (δ($CF_2$ benzyl) = − 108,7 ppm et − 107,3 ppm).

EXEMPLE 16

Dans un erlenmeyer, on place 25 ml de diméthylformamide, 7 ml d'eau, 5 ml d'aniline, 5 g de dithionite de sodium, 5 g de phosphate disodique et 9,4 g de trichloro-1,1,1 trifluoro-2,2,2 éthane. On agite à température ordinaire pendant 48 heures, puis les (dichloro-1′,1′ trifluoro-2′,2′,2′ éthyl)anilines sont entraînées à la vapeur.

Après extraction à l'éther et purification on obtient
2,2 g de (dichloro-1′,1′ trifluoro-2′,2′,2′, éthyl)-2 aniline
Rdt = 18% ; δF = − 69 ppm
0,6 g de (dichloro-1,1′ trifluoro-2′,2′,2′ éthyl)-4 aniline
Rdt = 5% ; δF = − 68,3 ppm

EXEMPLE 17

On procède comme à l'exemple 1 en remplaçant la pression en bromotrifluorométhane par une pression

de 1 bar d'iodopentafluoroéthane.

Après purification, on obtient :

— 4,4 g de pentafluoroéthyl-2 aniline ; Rdt = 21%
δF (benzyl) = – 113 ppm
— 3,5 g de pentafluoroéthyl-4 aniline ; Rdt = 16%
δF (benzyl) = – 113,6 ppm
— 0,5 g de bis-pentafluoroéthyl-2,6 aniline ; Rdt = 2%
δF (benzyl) = – 114 ppm
— 1,1 g de bis-pentafluoroéthyl-2,4 aniline ; Rdt = 3%
δF (benzyl) = – 113 ppm, – 114,6 ppm

EXEMPLE 18

Dans un flacon en verre épais on place 16,8 g d'hydroxyméthanesulfinate de sodium, 50 ml de diméthylformamide, 4 ml d'eau et 10 ml d'aniline. On fait le vide dans le flacon puis on y introduit 1 g de dioxyde de soufre. Le flacon est thermostaté à 65°C puis il est agité pendant 2 heures sous une pression de bromotrifluorométhane qui est maintenue entre 4 et 2 bars. Le flacon est ensuite ouvert et les trifluorométhylanilines sont entraînées à la vapeur. On obtient ainsi les trifluorométhyl-2 et 4 anilines.

EXEMPLE 19

On procède comme dans l'exemple 18 en remplaçant l'aniline, le diméthylformamide et le dioxyde de soufre par 50 ml de pyridine. Après 2 heures de réaction, les solides sont éliminés par filtration. L'analyse du résidu par résonance magnétique nucléaire du fluor et par chromatographie en phase gazeuse indique la formation de

trifluorométhyl-2 pyridine ; Rdt = 4% ; δF = – 61,7 ppm
trifluorométhyl-3 pyridine ; Rdt = 1% ; δF = – 64,4 ppm
trifluorométhyl-4 pyridine ; Rdt = 5% ; δF = – 67,7 ppm

EXEMPLE 20

On procède comme à l'exemple 19 en remplaçant l'hydroxyméthanesulfinate de sodium par l'hydroxyméthanesulfinate de zinc. On obtient ainsi les trifluorométhyl-2, -3 et -4 pyridines avec les rendements respectifs de 4%, 1% et 4%.

EXEMPLE 21

Dans un réacteur de 400 ml, on charge en solution 100 ml d'eau et 42,6 g de phosphate disodique (0,3 mole), puis on charge 6,7 g (0,03 mole) de dithionite de sodium (pureté 78,6%), le rapport substrat sur dithionite est égal à 10. Pour terminer, on charge en solution 150 g de diméthylformamide et 27,9 g d'aniline (0,3 mole).

Après fermeture du réacteur, on met sous vide (50 mm de Hg) puis on admet le trifluorobromométhane sous une pression de 13 bars.

On agite, chauffe jusqu'à 55°C et on maintient la pression à 13 bars.

La durée totale de l'essai est de 1 h 30. Après refroidissement et dégazage on décante la couche supérieure.

L'analyse RMN 19F ($CF_3CO_2H$) donne comme résultats :

— Rdt 17,6% de trifluorométhyl-2 aniline t = 15,5 ppm
— Rdt 9,1% de trifluorométhyl-4 aniline t = 17,5 ppm
— Rdt 0,4% de bistrifluorométhyl-2,6 aniline t = 14,5 ppm
— Rdt 0,9% de bistrifluorométhyl-2,4 aniline t = 14,7 ppm et 16,8 ppm

EXEMPLE 22

Avec la pyridine, on procède comme dans l'exemple 21, avec les mêmes rapports de réactifs et on obtient les trifluorométhylpyridines avec un Rdt de 1%.

Les différents isomères sont obtenus dans le rapport :

— 60% de trifluorométhyl – 2 pyridine tF = 15,5 ppm ($CF_3CO_2H$)
— 10% de trifluorométhyl – 3 pyridine tF = 13 ppm

— 30% de trifluorométhyl – 4 pyridine tF = 10 ppm.

EXEMPLE 23

Avec le pyrrole, on procède comme l'exemple 21, avec les mêmes rapports de réactifs. Le temps de réaction est de 3 h 45. On obtient le trifluorométhyl — 2 pyrrole avec un Rdt de 47,4%.

— $CF_3$ t = 47 ppm ($CF_3CO_2H$)
— H-3 t = 6,59 ppm (1H, d) (TMS)
— H-4 t = 6,21 ppm (1H, t) (TMS)
— H-5 t = 7,00 ppm (1H, d) (TMS)
— C-3 t = 108,5 ppm (TMS)
— C-4 t = 107,4 ppm
— C-5 t = 121,1 ppm.

EXEMPLE 24

Avec l'hydroxy-8 quinoléine on procède comme l'exemple 21, avec les mêmes rapports de réactifs. Le temps de réaction est de 3 heures. On obtient le trifluorométhyl — 5 hydroxy — 8 quinoléine avec un Rdt de 10,4%.

— $CF_3$ t = 20 ppm ($CF_3CO_2H$)
— H-2 t = 9,04 ppm (1H, d) (TMS)
— H-3 t = 7,82 ppm (1H, t)
— H-4 t = 8,49 ppm (1H, d)
— H-6 t = 7,97 ppm (1H, d)
— H-7 t = 7,24 ppm (1H, d)

EXEMPLE 25

Avec le résorcinol, on procède comme dans l'exemple 21, le rapport substrat sur le dithionite est égal à 5. Le temps de réaction est de 4 heures.

On obtient les dérivés trifluorométhyés avec un Rdt de 10%. Les isomères sont obtenus dans le rapport de 80% de trifluorométhyl- 4 dihydroxy — 1,3 benzène 19F t = 17,5 ppm ($CF_3CO_2H$) et de 20% de trifluorométhyl — 2 dihydroxy — 1,3 benzène 19F t = 23,4 ppm.

EXEMPLE 26

Avec le thiophéne, on procède comme dans l'exemple 21, le rapport substrat sur le dithionite est égal à 5. Le temps de réaction est de 4 heures 30.

On obtient deux isomères trifluorométhylés avec un Rdt de 10%. RMN 19F : δ = 6,8 ppm et δ = 7,2 ppm.

EXEMPLE 27

Avec le 4-hydroxybiphényle, on procède comme dans l'exemple 21, le rapport substrat sur le dithionite est égal à 5. Le temps de réaction est de 2 heures 30.

On obtient trois isomères trifluorométhylés avec un Rdt de 4,6%. RMN 19F : δ 16 ppm, δ = 11,4 ppm et δ = 21,8 ppm.

EXEMPLE 28

Avec le 4,4-dihydroxybiphényle, on procède comme dans l'exemple 21, le rapport substrat sur le dithionite est égal à 5. Le temps de réaction est de 2 heures 30.

On obtient deux isomères trifluorométhylés avec un Rdt de 3%. RMN 19F : δ = 16,13 ppm et δ = 11,70 ppm.

EXEMPLE 29

Avec le furanne, on procède comme dans l'exemple 21, le rapport substrat sur le dithionite est égal à 5. Le temps de réaction est de 4 heures.

On obtient deux isomères trifluorométhylés avec un Rdt de 3%. RMN 19F : δ = –2,50 ppm et δ = –1,78 ppm.

EXEMPLE 30

Avec l'hydroxy-4 quinoléine, on procède comme dans l'exemple 21, le rapport substrat sur le dithionite est égal à 5. Le temps de réaction est de 3 heures.

On obtient quatre isomères trifluorométhylés avec un Rdt de 5,8%. RMN 19F : δ = 8 ppm, δ = 15,5 ppm, δ = 16,2 ppm et δ = 17 ppm.

**Revendications**

1. Procédé de préparation de dérivés aromatiques perhalogénoalkylés caractérisé en ce que l'on met en présence, dans un solvant polaire, un dérivé aromatique, un dithionite ou un hydroxyméthanesulfinate alcalin, alcalino terreux ou métallique avec un halogénure de perfluoroalkyle ou un halogénure de perhalogénoalkyle trifluorométhylé en ce que le dérivé aromatique répond à la formule générale (I) :

$$Ar\,(R)_n \quad (I)$$

dans laquelle :
— Ar représente un radical aromatique mono ou polycyclique ou hétérocyclique,
— R représente au moins un substituant choisi parmi l'hydrogène, le chlore, le brome, les radicaux alkyles linéaires, ramifiés, cycliques saturés ou insaturés éventuellement substitués, éther, alcoxy éventuellement substitué, aryl éventuellement substitué, aryloxy, alkylthio, arylthio, amino, hydroxy, carboxylate, acyloxy, ester, amido, nitrile, acide,
— n est un nombre entier égal à 1, 2 ou 3

2. Procédé selon la revendication 1, caractérisé en ce que, dans la formule générale (I)
– Ar représente un radical aromatique monocyclique,
– R représente un radical amino ou hydroxy.

3. Procédé selon la revendication 1 caractérisé en ce que le dithionite alcalin, alcalino terreux ou métallique répond à la formule (III)

$$M(S_2O_4)_n \quad (III)$$

dans laquelle
n est égal à 1 ou 2 suivant la valeur du métal,
M représente le sodium ou le potassium.

4. Procédé selon la revendication 1 caractérisé en ce que l'hydroxyméthanesulfinate alcalin, alcalino-terreux ou métallique répond à la formule (IV)

$$(HOCH_2SO_2)_nM \quad (IV)$$

dans laquelle n et M ont la même signification que dans la formule (III)

5. Procédé selon la revendication 1 caractérisé en ce que l'halogénure de perfluoroalkyle ou de perhalogénoalkyle trifluorométhylé répond à la formule suivante

$$CF_3\text{-}A\text{-}X \quad (II)$$

dans laquelle
• X représente un halogène choisi parmi le brome, l'iode, ou le chlore et lorsque A représente une liaison covalente X est choisi parmi le brome et l'iode
• A représente une liaison covalente, un groupe $(CF_2)_n$ où n est un nombre entier compris entre 1 et 2, un groupe $—CCl_2—$.

6. Procédé selon la revendication 5 caractérisé en ce que l'halogénure de perfluoroalkyle ou de perhalogénoalkyle trifluorométhylé est choisi parmi le bromure de trifluorométhyle, les iodures de perfluoroalkyle, et le trifluoro-1,1,1 trichloro-2,2,2 éthane.

7. Procédé selon la revendication 1, caractérisé en ce que le solvant polaire est choisi parmi le formamide,

le diméthylformamide, le diméthylsulfoxyde, l'acétonitrile, l'hexaméthylphosphoramide, le diméthylacétamide, la N-méthylpyrolidone, le sulfolane.

8. Procédé selon la revendication 1, caractérisé en ce qu'on ajoute une base minérale.

9. Procédé selon la revendication 1 caractérisé en chaque le rapport molaire de la base au dérivé aromatique est compris entre 0,3 et 3.

10. Procédé selon la revendication 6 caractérisé en ce que la température de mise en oeuvre de la réaction est comprise entre 20 et 85° C et de préférence entre 45 et 75°C.

## Patentansprüche

1. Verfahren zur Herstellung von perhalogenalkylierten aromatischen Verbindungen, dadurch gekennzeichnet, daß man in einem polaren Lösungsmittel eine aromatische Verbindung, ein Alkali-, Erdalkali- oder Metalldithionit oder -hydroxymethansulfinat mit einem Perfluoralkylhalogenid oder einem trifluormethylierten Perhalogenalkylhalogenid zusammenbringt und daß die aromatische Verbindung der allgemeinen Formel (I)

$$Ar(R)_n \quad (I)$$

entspricht, in der:

— Ar für einen mono- oder polycyclischen aromatischen oder einen heterocyclischen Rest steht,

— R mindestens einen Substituenten bedeutet, der unter Wasserstoff, Chlor, Brom, den linearen, verzweigten, cyclischen, gesättigten oder ungesättigten und gegebenenfalls substituierten Alkylgruppen, Ether, gegebenenfalls substituiertes Alkoxy, gegebenenfalls substituiertes Aryl, Aryloxy, Alkylthio, Arylthio, Amino, Hydroxy, Carboxylat, Acyloxy, Ester, Amido, Nitril und Säure ausgewählt ist,

— n eine ganze Zahl von 1, 2 oder 3 ist.

2. Verfahren nach Anspruch 1, dadurch gekennzeichnet, daß in der allgemeinen Formel (I)

— Ar für einen monocyclischen aromatischen Rest und

— R für eine Amino- oder Hydroxygruppe steht.

3. Verfahren nach Anspruch 1, dadurch gekennzeichnet, daß das Alkali-, Erdalkali- oder Metalldithionit der Formel (III)

$$M(S_2O_4)_n \quad (III)$$

entspricht, in der

n 1 oder 2 ist, je nach der Wertigkeit des Metalls, und

M für Natrium oder Kalium steht.

4. Verfahren nach Anspruch 1, dadurch gekennzeichnet, daß das Alkali-, Erdalkali- oder Metallhydroxymethansulfinat der Formel (IV)

$$M(SO_2CH_2OH)_n \quad (IV)$$

entspricht, in der n und M die gleiche Bedeutung wie für die Formel (III) haben.

5. Verfahren nach Anspruch 1, dadurch gekennzeichnet, daß das Perfluoralkylhalogenid oder das trifluormethylierte Perhalogenalkylhalogenid der folgenden Formel

$$CF_3\text{-}A\text{-}X \quad (II)$$

entspricht, in der

— X ein Halogenatom, ausgewählt unter Brom, Iod oder Chlor, bedeutet, wobei, wenn A für eine kovalente Bindung steht, X unter Brom und Iod ausgewählt wird,

— A eine kovalente Bindung, eine Gruppe $(CF_2)_n$, in der n eine ganze Zahl von 1 oder 2 ist, oder eine Gruppe $—CCl_2—$ bedeutet.

6. Verfahren nach Anspruch 5, dadurch gekennzeichnet, daß das Perfluoralkylhalogenid oder das trifluormethylierte Perhalogenalkylhalogenid unter Trifluormethylbromid, den Perfluoralkyliodiden und 1,1,1-Trifluor-2,2,2-trichlorethan ausgewählt wird.

7. Verfahren nach Anspruch 1, dadurch gekennzeichnet, daß das polare Lösungsmittel unter Formamid, Dimethylformamid, Di-methylsulfoxid, Acetonitril, Hexamethylphosphoramid, Dimethylacetamid, N-Methylpyrrolidon und Sulfolan ausgewählt wird.

8. Verfahren nach Anspruch 1, dadurch gekennzeichnet, daß man eine anorganische Base zusetzt.

9. Verfahren nach Anspruch 1, dadurch gekennzeichnet, daß das Molverhältnis von Base zu aromatischer Verbindung im Bereich von 0,3 bis 3 liegt.

10. Verfahren nach Anspruch 6, dadurch gekennzeichnet, daß die Reaktionstemperatur im Bereich von 20 bis 85°C, vorzugsweise im Bereich von 45 bis 75°C liegt.

## Claims

1. Process for the preparation of perhaloalkylated aromatic derivatives, characterised in that an aromatic derivative and an alkali, alkaline-earth or other metal dithionite or hydroxymethanesulphinate are brought together with a perfluoroalkyl halide or a trifluoromethylated perhaloalkyl halide, in a polar solvent and in that the aromatic derivative corresponds to the general formula (I) :

$$Ar(R)_n \quad (I)$$

in which :

— Ar denotes a mono- or polycyclic or heterocyclic aromatic radical,

— R denotes at least one substituent chosen from hydrogen, chlorine, bromine and linear, branched, cyclic, saturated or unsaturated, optionally substituted alkyl radicals, and ether, optionally substituted alkoxy, optionally substituted aryl, aryloxy, alkylthio, arylthio, amino, hydroxy, carboxylate, acyloxy, ester, amido, nitrile and acid radicals, and

— n is an integer equal to 1, 2 or 3.

2. Process according to Claim 1, characterised in that, in the general formula (I)

— Ar denotes a monocyclic aromatic radical, and

— R denotes an amino or hydroxy radical.

3. Process according to Claim 1, characterised in that the alkali, alkaline-earth or other metal dithionite corresponds to the formula (III)

$$M\,(S_2O_4)_n \quad (III)$$

in which

n is equal to 1 or 2, depending on the valency of the metal, and

M denotes sodium or potassium.

4. Process according to Claim 1, characterised in that the alkali, alkaline-earth or other metal hydroxymethanesulphinate corresponds to the formula (IV)

$$(HOCH_2SO_2)_nM \quad (IV)$$

in which n and M have the same meaning as in the formula (III).

5. Process according to Claim 1, characterised in that the perfluoroalkyl or trifluoromethylated perhaloalkyl halide corresponds to the following formula

$$CF_3\text{-}A\text{-}X \quad (II)$$

in which

X denotes a halogen chosen from bromine, iodine or chlorine and, when A denotes a covalent bond,

X is chosen from bromine and iodine, and A denotes a covalent bond, a $(CF_2)_n$ group, where n is an integer between 1 and 2, or a $-CCl_2-$ group.

6. Process according to Claim 5, characterised in that the per fluoroalkyl or trifluoromethylated perhaloalkyl halide is chosen from trifluoromethyl bromide, perfluoroalkyl iodides, and 1,1,1-trifluoro-2,2,2-trichloroethane.

7. Process according to Claim 1, characterised in that the polar solvent is chosen from formamide, dimethylformamide, dimethyl sulphoxide, acetonitrile, hexamethylphosphoramide, dimethylacetamide, N-methylpyrrolidone, and sulpholane.

8. Process according to Claim 1, characterised in that an inorganic base is added.

9. Process according to Claim 1, characterised in that the molar ratio of the base to the aromatic derivative is between 0.3 and 3.

10. Process according to Claim 6, characterised in that the temperature at which the reaction is implemented is between 20 and 85°C and preferably between 45 and 75°C.